(19) Europäisches Patentamt

European Patent Office

Office européen des brevets

(11) **EP 0 625 342 B1**

(12) **EUROPÄISCHE PATENTSCHRIFT**

(45) Veröffentlichungstag und Bekanntmachung des Hinweises auf die Patenterteilung:
**12.08.1998 Patentblatt 1998/33**

(51) Int. Cl.⁶: **A61F 5/01**, C08K 7/02, B23B 27/12

(21) Anmeldenummer: **94107431.2**

(22) Anmeldetag: **13.05.1994**

(54) **Formkörper, insbesondere für orthopädische Zwecke, Verfahren zu seiner Herstellung, Vorrichtung zur Durchführung des Verfahrens und Verwendung des Formkörpers**

Mouldable material, particularly for orthopedic applications, method of its production, device for performing this method, and utilisation of the material

Matériau moulable, particulièrement pour des applications orthopédiques, procédé pour sa fabrication, dispositif pour mettre en oeuvre ce procédé et utilisation du matériau

(84) Benannte Vertragsstaaten:
**AT CH DE ES FR GB IT LI NL SE**

(30) Priorität: **18.05.1993 DE 4316442**

(43) Veröffentlichungstag der Anmeldung:
**23.11.1994 Patentblatt 1994/47**

(73) Patentinhaber:
• **Küschall, Rainer**
  **CH-4123 Allschwil (CH)**
• **Heckel, Gerd**
  **71159 Mötzingen (DE)**

(72) Erfinder:
**Heckel, Gerd, Dipl.-Ing. (FH)**
**D-71159 Mötzingen (DE)**

(74) Vertreter:
**Späth, Dieter, Dipl.-Ing. et al**
**Klocke - Späth - Neubauer**
**Patentanwälte,**
**Kappelstrasse 8**
**72160 Horb (DE)**

(56) Entgegenhaltungen:
DE-A- 3 304 537          GB-A- 2 151 543
NL-A- 8 900 125          US-A- 4 908 402
US-A- 5 240 773

• **PATENT ABSTRACTS OF JAPAN, Band 81, Nr. 10 (C-224), 23 Mai 1984**

**Beschreibung**

Die Erfindung betrifft einen Formkörper, insbesondere für orthopädische Geräte, ein Verfahren zur Herstellung dieses Formkörpers, insbesondere für orthopädische Zwecke, eine Vorrichtung zur Durchführung des genannten Verfahrens und eine Verwendung des genannten Formkörpers.

Zur Herstellung von orthopädischen Geräten werden seit langem Materialien verwendet, bei welchen eine Matrix aus Kunststoff durch eingelegte Fasern verstärkt ist. Kunststoffe eignen sich aus verschiedenen Gründen besser als die früher üblichen Materialien, wie zum Beispiel Gips, Leder oder Metalle, da sie leichter, dauerhafter, feuchtigkeitsunempfindlicher, hautverträglicher und einfacher in die gewünschte Form bringbar sind.

Im allgemeinen wurden bisher als Werkstoffe für die Matrix von orthopädischen Geräten duroplastische Harze verwendet. Der Nachteil dieser Werkstoffe besteht darin, dass sie sich zwar problemlos bei der Herstellung von orthopädischen Teilen -mit und ohne Fasern- in die für das orthopädische Gerät gewünschte Form bringen lassen, dann aber nach der Aushärtung kein weiteres Mal plastisch verformbar sind. Solche orthopädische Geräte sind damit oft nicht optimal, weil es nicht möglich ist, ihre Form nach einem versuchsweisen Gebrauch zu perfektionieren oder sie im Laufe der Zeit anatomischen Veränderungen oder neuen Bedürfnissen anzupassen.

Zur Herstellung von orthopädischen Geräten, die sich in der erwünschten Weise nachverformen lassen, verwendete man daher auch ein sandwichartig aufgebautes Material, das Aussenschichten aus einem duroplastischen Kunststoff aufweist, zwischen welchen sich ein Kern aus einem thermoplastischen Kunststoff befindet. Bei der Formgebung dieses Materials werden die duroplastischen Aussenschichten nur elastisch verformt, während die Verformung des Kerns eine plastische ist. Der plastisch verformte Kern hält dann die elastisch verformten Aussenschichten in vorgespannter Lage fest. Eine Nachverformung ist jederzeit möglich, indem man den Kern nochmals erwärmt und plastisch verformt, wobei natürlich die Elastizitätsgrenzen der Aussenschichten berücksichtigt werden müssen.

Orthopädische Geräte aus diesem sandwichartigen Material weisen aber ebenfalls einen wesentlichen Nachteil auf. Zu den durch die Formgebung verursachten Spannungen in den Aussenschichten addieren sich weitere, durch Beanspruchungen beim Gebrauch der orthopädischen Geräte verursachte Spannungen. Die Summe dieser Spannungen darf natürlich die zulässige Spannung des Materials nicht überschreiten, was zur Folge hat, dass nur verhältnismässig geringe Gebrauchsspannungen zugelassen werden können.

Es ist ferner zu berücksichtigen, dass die Materialschichten infolge ihrer sandwichartigen Konfiguration einen Schubverbund hoher Steifigkeit mit einem hohen Elastizitätsmodul E bilden. Und da im elastischen Bereich generell gilt, dass Deformationen, d.h. Verschiebungen f dem Quotienten aus Spannung/Elastizitätsmodul entsprechen, haben hohe Elastizitätsmodule ohnehin kleine Verschiebungen zur Folge. Ausserdem gilt, dass so wie die Summe aus Formgebungsspannung und Gebrauchsspannung die zulässige Spannung nicht überschreiten darf, auch die Summe aus Formgebungsverschiebung f(D) und Gebrauchsverschiebung f(G) die zulässige Verschiebung F(ZU) nicht überschreiten darf. Für die gerade noch zulässige Formgebungsverschiebung f(D) gilt deshalb

$$f(D) = F(ZUL) - f(G)$$

Aufgrund dieser Beziehungen der Deformationen resultiert -je nach der notwendigen Gebrauchsverschiebung f(G)- nur eine geringe Formgebungsverschiebung f(D), was je nach Art des orthopädischen Gerätes unerwünscht sein kann

Es wurde daher versucht, zur Herstellung orthopädischer Geräte faserverstärkte teilkristalline thermoplastische Kunststoffe zu verwenden, Im Gegensatz zu den sandwichartigen Materialien lassen teilkristalline thermoplastische Kunststoffe eine genügende Deformation beim Gebrauch zu. Ausserdem lassen sie sich - im Gegensatz zu den oben erwähnten duroplastischen Kunststoffen- wiederholt plastisch verformen, wenn sie auf die entsprechende Temperatur erwärmt werden. Sie weisen aber zwei Nachteile auf, die ihre Verwendung insbesondere für orthopädische Geräte problematisch machen. Erstens müssen die teilkristallinen thermoplastischen Kunststoffe nach ihrer Verformung verpresst werden, was bei orthopädischen Geräten kaum möglich ist; und zweitens ist der Temperaturbereich, in welchem sich diese Kunststoffe thermoplastisch verhalten, sehr eng. Sinkt die Temperatur unter diesen Bereich, so ist der Kunststoff nicht mehr plastisch verformbar, steigt die Temperatur über diesen Bereich, so schmilzt das Material. Die Obergrenze und die Untergrenze des Temperaturbereichs unterscheiden sich nur um etwa 5° C. Es ist leicht einzusehen, dass das Einhalten dieses Temperaturbereichs kaum möglich ist, so dass das Material nicht in einfacher Weise plastisch verformt werden kann.

Im Pultrusionsverfahren kontinuierlich hergestellte bevorzugt band-, schnur-, stangen- oder profil förmige faserverstärkte Strukturen mit einer Matrix aus thermoplastischem Polymer und wenigstens 30 Vol.% Verstärkungsfasern, die in der Struktur längsausgerichtet sind, wobei die Verstärkungsfasern mit 2 bis 100 mm die gleiche Länge aufweisen, wie die Strukturen, beansprucht die EP 0 056 703 A1. Dabei sind band- und streifenartige Gebilde bevorzugt, die für Gewebe verwendet werden können. Als Polymeres wird unter vielen anderen auch PMMA genannt.

Die US 4,908,402 beschreibt mit 5 bis 50 Gew.% Kohlefaser verstärkte Harzzusammensetzungen für Formkörper und Extrusionsprodukte, wobei das Harz ein Polymeres mit einer ringförmigen Grundstruktur aus heterocyclischen Kettenbausteinen ist, die als wesentlich für die gesuchten Eigenschaften bezeichnet wird. Die Verstärkungsfasern sind keine Filamente, sondern sogenannte Kurzschnitt-Stapelfasern.

Ausgehend von diesem Stand der Technik werden die Aufgaben der Erfindung somit darin gesehen,

a) einen Formkörper der eingangs genannten Art zu schaffen, welcher leicht verformbar und in einfacher Weise nachverformbar ist und welcher eine genügende Deformation zulässt;

b) ein Verfahren zur Herstellung eines solchen Formkörpers vorzuschlagen;

c) eine Vorrichtung zur Durchführung des genannten Verfahrens zu beschreiben; und

d) eine Verwendung des genannten Formkörpers aufzuzeigen.

Erfindungsgemäss werden diese Aufgaben gelöst

a) für den Formkörper durch die Merkmale des Patentanspruchs 1;

b) für das Verfahren: durch die Merkmale des Patentanspruchs 6;

c) für die Vorrichtung: durch die Merkmale des Patentanspruchs 17 und

d) für die Verwendung: durch die Merkmale des Patentanspruchs 21.

Vorteilhafte Weiterbildungen werden durch die jeweiligen weiteren Patentansprüche beschrieben.

Der erfindungsgemässe Formkörper besteht aus einer Matrixschicht aus in einfacher Weise verformbarem amorphen, thermoplastischen Kunststoff, der sich nach jeweiliger Erwärmung beliebig oft nachverformen lässt, und aus darin in Schichtform eingebetteten Faserbahnen, die mit dem Kunststoff imprägniert sind bzw. diesen verstärken.

Der erzielte Fortschritt besteht darin, dass man mit dem neuen Formkörper orthopädische Geräte herstellen kann, die beim Gebrauch eine genügende elastische Deformation zulassen und die in einfacher Weise perfekt anpassbar sind, da man die ursprüngliche Formgebung nach einer versuchsweisen Verwendung durch eine Nachverformung verbessern kann; ausserdem lässt sich die Lebensdauer von orthopädischen Geräten durch die Verwendung des neuen Formkörpers erhöhen, weil man wegen der Nachverformbarkeit des Formkörpers Geräte an anatomische Veränderungen anpassen kann, statt sie, wie früher nötig, durch neue Geräte zu ersetzen. Die Formgebung kann entweder sofort bei der Herstellung mittels in-situ-Technik oder aber nach einem Zwischenschritt aus einer Halbzeugform, beispielsweise aus einer Platte oder einem Vorformling erfolgen.

Ein geeigneter amorpher thermoplastischer Werkstoff für die Matrixschichten ist Polymethylmethacrylat (PMMA), das sich bei Erwärmung auf 170 ° C bis 180 ° C nachverformen lässt. Diese Temperatur lässt sich mit jeder beliebigen Wärmequelle, am besten mit einem Heissluftföhn oder einem Wärmeschrank erzeugen.

Der Formkörper ist plattenförmig. Er kann eine einzige Matrixschicht mit einer einliegenden Schicht von Faserbahnen aufweisen. Je nach der vorgesehenen Beanspruchung kann es aber vorteilhaft sein, den Formkörper aus mehreren Matrixschichten aufzubauen; meist sind alle der zusätzlichen Matrixschichten mit Faserbahnen verstärkt. Die mit den schichtweise eingebetteten Faserbahnen verstärkten Matrixschichten bilden einen laminatartigen Formkörper, wobei sie ihren angrenzenden Bereichen ineinander übergehen, wie im Laufe'der Beschreibung später verdeutlicht wird.

Die Faserbahnen können je nach der vorgesehenen Verwendung des Formkörpers als Gelege, Gestricke, Gewirke, Gewebe oder Netz verwendet werden; insbesondere bei Gelegen und Geweben können auch die im wesentlichen in parallelen Ebenen befindlichen Fasern verschiedener Matrixschichten verschieden gerichtet sein. Am besten eignen sich Faserbahnen in leicht zuschneidbarer Form. Wird der Formkörper nur teilweise hoch beansprucht, so können auch nur einzelne Bereiche der Matrixschichten mit Faserbahnen belegt werden. Die Faserbahnen können dabei beanspruchungsgerecht orientiert werden. Wird z.B. der Formkörper vorwiegend in einer Richtung beansprucht, so verwendet man entsprechend orientierte Faserbahnen. Benötigt man aber einen in Plattenrichtung des Materials allseitig beanspruchbares Formkörper, so werden die Fasernbahnen entsprechend ungerichtet verwendet, wodurch man einen quasiisotropen Formkörper erhält. Die Faserbahnen aller Schichten werden durch den Kunststoff der Matrixschichten imprägniert und zusammengehalten.

Für den neuen Formkörper eignen sich Faserbahnen aus sämtlichen Arten und Mischungen handelsüblicher Fasern, wie Kohlenstoff-Fasern, Glasfasern und Keramikfasern. Sehr geeignet und häufig benutzt sind Kohlenstoff-Fasern. Durch die Verwendung verhältnismässig steifer Fasern erhält man einen Formkörper, mit welchem man einen gewissen Memory-Effekt erzielen kann; dies bedeutet, dass ein verformtes Teil unter Wärmeeinwirkung wieder in seine ursprüngliche Form zurückkehrt, was unter anderem zur Korrektur falscher Verformungen nützlich sein kann.

Im Hinblick auf den Schutz des Formkörpers bei Transport und Lagerung sowie im Hinblick auf die Verarbeitung, insbesondere durch Kleben, des Formkörpers hat es sich als besonders günstig erwiesen, für eine oder beide Aussenflächen des Formkörpers eine Deckschicht vorzusehen, die an der angrenzenden Matrixschicht haftet. Wie weiter unten beschrieben wird, ist eine solche Deckschicht im Hinblick auf die Herstellung des Formkörpers bevorzugt durchlässig.

Die Deckschicht ist im allgemeinen flexibel und wird zum Beispiel durch eine perforierte Folie oder ein Kunststoffgewebe gebildet.

Das Verfahren zur Herstellung des neuen Formkörpers umfasst die folgenden Schritte:

- Evakuieren eines Gemischs aus den unpolymerisierten Ausgangsstoffen des Polymehtylmethacrylats:
- Zugabe des Gemischs der Ausgangsstoffe auf oder in eine Reaktor-Form,
- Auflegen einer Faserbahn auf das Gemisch,
- Egalisieren und Verdichten des Gemischs zu einer ersten Matrixschicht,
- wahlweises Wiederholen von mindestens einzelnen dieser Schritte für weitere Matrixschichten,
- Aufbringen einer ablösbaren durchlässigen Deckschicht auf mindestens eine Aussenfläche der mindestens einen Matrixschicht,
- Druckverdichten
- Polymerisation der Monomeren in der mindestens einen Matrixschicht,

wobei der Formkörper in der Reaktor-Form nach der Reaktorimprägnierung der Faserbahn mit den noch niederviskosen unpolymerisierten Ausgangsstoffen durch deren Polymerisation in situ gebildet wird und wobei die erste Matrixschicht und die weiteren Matrixschichten mindestens teilweise ineinander übergehen und ein Laminat bilden..

Das erfindungsgemässe Verfahren ist in einfacher Weise, bevorzugt diskontinuierlich, durchführbar. Man vermischt die unpolymerisierten Ausgangsstoffe des Kunststoffs für die Matrixschicht und bringt sie als zähflüssiges Gemisch in bzw. auf eine Form; auf die so entstehende Matrixschicht werden die Faserbahnen gelegt; schliesslich erfolgt die Polymerisierung des Gemischs während mehrerer Stunden zum Kunststoff bzw. zur Matrixschicht, bei geeigneter Temperatur und in einer sauerstoffhaltigen Umgebung, im allgemeinen Luft. Das schon erwähnte PMMA eignet sich insbesondere deshalb, weil es bei einer Temperatur zwischen etwa 20 ° C und 25 ° C polymerisiert, d.h. also bei einer üblichen Raumtemperatur, so dass bei Umgebungstemperatur gearbeitet und die Form weder geheizt noch gekühlt werden muss.

Einen besonders guten und gleichmässigen Formkörper erhält man, wenn man die Matrixschicht nach dem Auflegen der Faserbahnen, aber vor bzw. zu Beginn ihrer Polymerisation egalisiert und verdichtet. Dabei werden die Faserbahnen in die Matrixschicht gedrückt bzw. das noch nicht polymerisierte zähflüssige Gemisch dringt durch die Zwischenräume der Faserbahnen an deren Oberseite, so dass die Faserbahnen schliesslich in den Kunststoff der Matrixschicht eingebettet und anderseits dadurch in ihrer Konfiguration gehalten sind.

Um einen Formkörper zu erhalten, der grösseren Beanspruchungen gewachsen ist, kann man auf die erste polymerisierende Matrixschicht weiteres zähflüssiges Gemisch aus den unpolymerisierten Ausgangsstoffen des Kunststoffes, gegebenenfalls mit eingebetteten Faserbahnen, aufbringen, das anschliessend mit einer ersten Schicht, zu einer Matrix polymerisiert. Man erhält so einen laminatartigen Formkörper, bei welchem aber die einzelnen Schichten nicht nur aneinander haften, sondern infolge ihrer praktisch simultanen Polymerisation in ihren Grenzbereichen ineinander übergehen, so dass keine Schichtgrenzen und damit keine Gefahr einer Delamination besteht. Natürlich lassen sich auf diese Weise beliebige weitere Matrixschichten erzeugen, bis der Formkörper die erforderliche Stärke erreicht.

Auch diese weiteren Matrixschichten werden im allgemeinen - wie weiter oben mit Bezug auf die erste Matrixschicht beschrieben - mit Faserbahnen belegt, egalisiert und verdichtet, so dass die Faserbahnen im Kunststoff eingebettet sind. Um einen allseits gleich beanspruchbaren, quasiisotropen Formkörper zu erhalten, können wie weiter oben beschrieben, die Faserbahnen verschiedener Matrixschichten in verschiedener Richtung angeordnet werden.

Wie schon erwähnt werden alle Schichten der Faserbahnen durch den Kunststoff zusammengehalten.

Nach dem schichtweisen Aufbau des Formkörpers kann unter Druck nochmals eine Verdichtung vorgenommen werden. Vorher kann der Formkörper, wie weiter oben ausgeführt wurde, mit einer durchlässigen, an ihm haftenden Deckschicht versehen werden, welche vor der Verarbeitung des Formkörpers seine Oberfläche schützt. Diese mit Vorteil flexible Deckschicht, beispielsweise ein Kunststoffgewebe oder eine perforierte Folie, wird vor bzw. zu Beginn der Polymerisation auf die äusserste Matrixschicht gelegt und unter Druck mit ihr verbunden. Der für die Polymerisation notwendige Sauerstoff wird der Luft entnommen, die in der Deckschicht, d.h. zwischen den Gewebefasern oder in den Perforationen, vorhanden ist.

Obwohl durch die durchlässige Deckschicht im allgemeinen genügend Sauerstoff zum Polymerisationsbereich Zutritt hat, kann es sich bei hohem Sauerstoffbedarf als notwendig erweisen, den Druck, der etwa 0.1 bis 0.5 bar betragen kann, auf die oberste Schicht des Formkörpers über eine weitere durchlässige, waben- oder gitterartige Zwischenschicht auszuüben, in deren freien Räumen weiterer Sauerstoff vorhanden ist. Bezüglich der Sauerstoffzufuhr bei der Verdichtung des Formkörpers unter Druck kann die Verwendung einer solchen gitter- oder wabenartigen Zwischenschicht als Hilfsmittel bei der Herstellung des Formkörpers auch anstelle der mit dem Formkörper zu verbindenden Deckschicht vorgesehen sein. Mittels der Zwischenschicht lässt sich auch eine Struktur auf der Aussenfläche des Formkörpers erzeugen.

Um ein Haften des fertigen Formkörpers an der Reaktor-Form zu verhindern, wird vorteilhaft vor dem Einfüllen des Gemischs ein Trennmittel in der Reaktor-Form verteilt. Gelangt bei der Herstellung von Formkörpern ohne Deckschicht eine gitter-oder wabenartige Zwischenschicht direkt auf die oberste Matrixschicht, so empfiehlt es sich, zur Verhinderung des Anhaftens von polymerisierendem Gemisch bzw. Kunststoff auch dort ein Trennmittel anzuwenden, ebenso bei allen weiteren Werkzeugen, die mit dem polymerisierenden Gemisch in Berührung kommen.

Die weiter oben beschriebene Egalisierung und Verdichtung jeder einzelnen Matrixschicht hat auch zur Folge, dass im Gemisch vorhandene Luftblasen verdrängt werden, welche sich sonst im fertigen Formkörper als Hohlräume oder poröse Bereiche manifestieren würden.

Weit wirkungsvoller ist es aber, wenn zu diesem Zweck das Gemisch vor dem Einbringen in bzw. auf die Reaktor-Form evakuiert und somit praktisch vollständig entgast wird. Die Vorrichtung zur Durchführung des beschriebenen Verfahrens besteht aus eimer Reaktor-Form zur Aufnahme des zähflüssigen Gemisches der unpolymerisierten Ausgangsstoffe, einer Einrichtung zum Egalisieren und Verdichten dieser Ausgangsstoffe und einem äusseren Druckkörper.

Je nach der gewünschten Konfiguration des herzustellenden Materials kann die Reaktor-Form eben oder gewölbt sein oder auch eine kompliziertere geometrische Form aufweisen; im allgemeinen wird ebener Formkörper hergestellt, so dass man eine ebenfalls ebene, plattenartige Reaktor-Form benützt.

Die Einrichtung zum Egalisieren und Verdichten besitzt eine Leiste, die im wesentlichen komplementär zur Reaktor- Form -also gerade oder gebogen- ausgebildet ist und die, anliegend an die jeweils zuletzt eingebrachte Matrixschicht, längs der Form respektive über die Form verschiebbar ist.

Einen besonders guten Effekt erreicht man, wenn der zur Berührung der Matrixschicht vorgesehene Rand der Leiste flexibel mit der Leiste verbunden ist oder aus einem flexiblen Formkörper, beispielsweise aus einer elastischen Lippe oder einer länglichen Bürste, besteht.

Zur Ausübung des Druckes auf die oberste Matrixschicht bzw. auf die durchlässige Deckschicht kann die Vorrichtung einen Druckkörper aufweisen, der komplementär zur Reaktor-Form bzw. zum Formkörper ausgebildet ist. Im häufigsten Fall, d.h. zur Erzeugung eines ebenen Formkörpers, ist der Druckkörper eine Platte, die quer zum Formkörper verschiebbar und auf den Formkörper pressbar ist. Obwohl man im allgemeinen einen den ganzen entstehenden Formkörper abdeckenden Druckkörper verwendet, kann man -insbesondere bei der Herstellung von einem gewölbten Formkörper-, auch mit einem kleineren Druckkörper arbeiten, der jeweils nur einen Bereich des Formkörpers abdeckt, und mehrmals an den Formkörper angepresst wird, so dass alle Bereiche des Formkörpers nacheinander verdichtet werden.

Die Vorrichtung kann ausserdem eine waben-oder gitterartige Zwischenschicht aufweisen, die sich unter dem Druckkörper auf den Formkörper legen lässt. Die Wirkung dieser Zwischenchicht wurde bereits beschrieben. Eine geeignete Wabenweite kann zum Beispiel etwa 0.5 cm betragen. Ein solche Zwischenschicht kann auch zur Erzeugung einer geeigneten Oberflächenstruktur des Formkörpers dienen.

Zwischen der waben- oder gitterförmigen Zwischenschicht und dem Druckkörper kann eine Kunststoffplatte vorgesehen sein.

Die Verwendung des neuen Formkörpers zur Herstellung orthopädischer Geräte erfolgt in der Weise, dass das Formkörper soweit erwärmt wird, dass er plastisch verformbar ist, was bei dem erwähnten PMMA bei etwa 170 ° C bis 180 ° C der Fall ist. Anschliessend erfolgt die erforderliche Formgebung des Formkörpers sowie seine Weiterverarbeitung, beispielsweise seine Verbindung mit anderen Bestandteilen des orthopädischen Gerätes. Stellt man nach der Formgebung des Formkörpers oder bei einer versuchsweisen Verwendung des orthopädischen Gerätes fest, dass eine Nachverformung notwendig ist, so lässt sich der Formkörper in einfacher Weise nochmals auf die erwähnte Temperatur erwärmen, wozu im allgemeinen ein Heissluftföhn reicht, worauf man die Nachverformung durchführen kann. In gleicher Weise kann eine Nachverformung stattfinden, wenn das orthopädische Gerät einer anatomischen Veränderung angepasst werden muss, die sich im Laufe der Zeit ergeben hat.

Der neue Formkörper lässt sich ohne Schwierigkeiten zu gewölbten Flächen verformen; der kleinstmögliche Wölbungsradius wird dabei weitgehend von der Steifigkeit der Fasern bestimmt. Eine sphärische Verformung des Formkörpers ist aber nur in sehr begrenztem Ausmass möglich. Zur Herstellung von sphärisch gekrümmten Flächen ist es daher im allgemeinen nötig, den Formkörper mit Ausschnitten zu versehen. Um festzustellen, ob und in welchen Abmessungen solche Ausschnitte anzubringen sind, kann versuchsweise eine Faserbahn derselben Art, wie es im Formkörper einliegend verwendet wird, der gleichen Formgebung unterworfen werden, die für den Formkörper vorgesehen ist. Lässt sich diese Faserbahn im erforderlichen Ausmass verformen, so kann auch der Formkörper im praktisch gleichen Ausmass verformt werden. Eine solche, nicht in Kunststoff eingebettete Faserbahn, kann also gewissermassen als Schnittmuster für den Formkörper benutzt verwendet werden,

Wird ein Formkörper benützt, der mit einer Deckschicht versehen ist, so muss diese Deckschicht vor der Formgebung des Formkörpers entfernt werden, was in einfacher Weise durch Wegziehen oder Wegreissen möglich ist. Das Entfernen der Deckschicht wird erleichtert, wenn sie einen kleinen über die Matrixschicht hinausragenden Lappen aufweist, der mit einem Instrument oder von Hand ergriffen werden kann. Nach dem Entfernen der Deckschicht erhältman einen Formkörper mit einer Oberfläche, die frei von Verschmutzungen und kleinen Beschädigungen ist. Ausserdem ist diese Oberfläche leicht aufgerauht, so dass sie ohne Schwierigkeiten mit anderen Bestandteilen des orthopädischen Gerätes durch Kleben verbunden werden kann, ohne dass sie vor dem Auftragen von Klebstoff angeschliffen oder sonstwie aufgerauht werden muss. Natürlich kann der Formkörper auch mittels mechanischer Befestigungsmittel mit anderen Bestandteilen orthopädischer Geräte, wie zum Beispiel mit anderen Kunststoffen oder Metallen, verbunden werden.

In der Folge werden an einem Ausführungsbeispiel und unter Bezugnahme auf die Zeichnung einige Aspekte der Erfindung weiter erläutert, Es zeigt:

Fig. 1      das Gemisch der unpolymerisierten Ausgangsstoffe des Kunststoffs in einer Vakumieranlage, in einem vereinfachten Schaubild;

Fig. 2      einen Teil einer Vorrichtung zur Herstellung des neuen Formkörpers, in welcher auch Teile des Formkörpers sichtbar sind, in einem vereinfachten Schaubild;

Fig. 3      einen weiteren Teil der Vorrichtung der **Fig. 2,** in welcher der Formkörper sichtbar ist, in einem vereinfachten Schaubild;

Fig. 4      der neue Formkörper, in einer Schnittdarstellung.

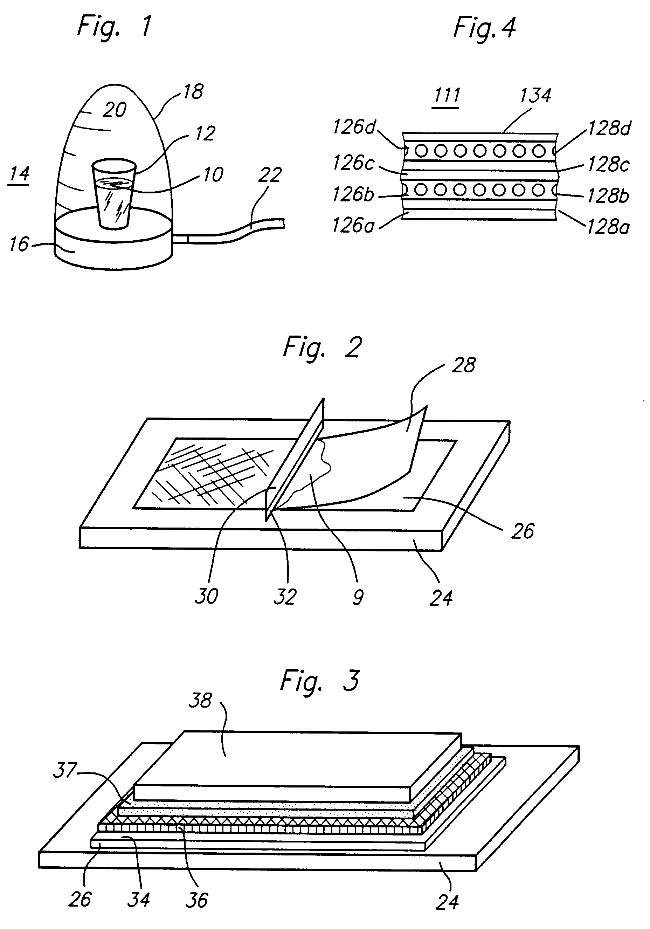

Gemäss **Fig. 1** befindet sich ein Gemisch **10** aus den unpolymerisierten Ausgangsstoffen des Kunststoffes, der zur Bildung der Matrixschichten vorgesehen ist, in einem Behälter **12,** welcher in einer Vakumieranlage **14** steht. Diese Vakumieranlage **14** weist eine Grundplatte **16** und eine Glocke **18** auf, welche zusammen einen Raum **20** dichtend begrenzen. An diesen Raum **20** ist eine Leitung **22** angeschlossen, über welche bei der Evakuierung des Raums **20** mittels einer nicht dargestellten Pumpe, das abgezogene Gas wegströmt.

Zur Herstellung des bevorzugt verwendeten Polymethylmethacrylat (PMMA) werden die folgenden Ausgangsstoffe in folgender Zusammensetzung verwendet:

Methylmethacrylat
100 Gewichtseinheiten
Milchsäure
0.5 Gewichtseinheiten
Cyclohexanonperoxid -Gemisch, z.B. Cyclonox LNC :
0.5 Gewichtseinheiten
Vanadiummonobutylphosphit, z.B. VN2
0.5 Gewichtseinheiten

Cyclonox LNC und VN2 sind Produkte der AKZO Chemicals, es können aber auch polymerisationsauslösende Reaktionsbestandteile anderer Hersteller verwendet werden.

Milchsäure, Cyclonox LNC und VN2 werden nacheinander in das Methylmethacrylat eingerührt. Da das Methylmethacrylat keine Newton' sche sondern eine strukturviskose Flüssigkeit ist, wird beim Mischvorgang sehr viel Luft in das Gemisch eingebracht, welche im fertigen Formkörper die Bildung von Lunkern oder porösen Bereichen zur Folge hätte, weshalb die Mischung, wie oben beschrieben, vor ihrer weiteren Verwendung in der Vakumieranlage **14** evakuiert wird.

**Fig. 2** zeigt eine plattenartige Reaktor-Form **24;** es kann sich dabei um eine einfache Kunststofform handeln, wobei für viele Fälle auch verlorene Schaumstofformen ausreichend sind.

Die Reaktor-Form **24** wurde vor dem Aufbringen des Gemisches **10** mit einem nicht eingezeichneten Trennmittel behandelt, um ein Anhaften des Gemischs **10** bzw. des daraus entstehenden Kunststoffs an der Reaktor-Form **24** zu verhindern. Geeignet dafür sind handelsübliche Trennmittel aus der Faserverbundtechnik.

Sodann wurde auf die Reaktor-Form **24** das Gemisch **10,** das zur Bildung einer Matrixschicht **26** dient, aufgebracht. **Fig. 2** zeigt, wie anschliessend Faserbahn **28** auf die Matrixschicht **26** gelegt wird. Die Hauptrichtung der Faserbahnen **28** wird bestimmt durch die beim Gebrauch des entstehenden Materials aufzunehmenden Spannungen. Bei den Fasern der Faserbahn **28** handelt es sich um Kohlenstoffasern, es können aber auch Fasern anderer Art verwendet werden, und zwar als Gelege, Gestricke, Gewirke oder Gewebe.

Von der Einrichtung zum Egalisieren und Verdichten des polymerisierenden Gemisches **10** ist in Fig. 2 lediglich eine Leiste **30** dargestellt, deren das Gemisch **10** berührender Rand durch eine flexible Lippe **32** gebildet wird. Die Leiste **30** wird unter leichtem Druck über die Faserbahn **28** verschoben wird, was zur Folge hat, dass eine gewisse Menge 9 des Gemisches **10** an die Oberseite der Faserbahn **28** gelangt. Auf diese Weise werden die Fasern der Faserbahn **28** in die Matrixschicht **26** eingebettet und vom noch nicht polymerisierten Kunststoff imprägniert.
Der Vorgang zur Erzeugung solcher Schichten wird so oft wiederholt, bis man aus der erwünschten Anzahl Matrixschichten **26,** jeweils mit eingelegten Faserbahnen **28,** den Formkörper **11** -allerdings ohne Deckschicht- aufgebaut hat, wobei der Kunststoff die FaserBahn-Schichten zusammenhält.

**Fig. 3** zeigt, wie anschliessend eine durchlässige Deckschicht **34** in Form eines Kunststoffgewebes auf die oberste der Matrixschichten **26** gelegt wird. Diese Deckschicht **34** ist die letzte bzw. oberste der Schichten, aus denen der Formkörper **11** besteht.

Sodann wird eine Verdichtung des Formkörpers **11** unter Druck vorgenommen. Zu diesem Zweck wird vorerst eine waben- oder gitterartige Zwischenschicht **36** auf den Formkörper **11** gelegt, die beispielsweise aus Aluminium oder Aramid gefertigt und etwa 1 cm dick ist, und an welche sich oben eine Schaumstoffplatte **37** anschliesst. Diese Platte schützt eine als Druckkörper wirkende Stahlplatte **38** vor Beschädigungen durch die waben- oder gitterartige Zwischenschicht **36** und gewährleistet, dass der Druck gleichmässig ausgeübt wird. Durch den Druck der Stahlplatte **38** wird der Formkörper **11** verdichtet, und gleichzeitig erzielt man damit die Haftung der Deckschicht **34** auf der obersten der Matrixschichten **26**. Wichtig ist, dass dank der Durchlässigkeit der Deckschicht **34** und der Zwischenschicht **36** dabei Sauerstoff aus der Umgebungsluft an den Formkörper gelangen kann, da dieser für die Polymerisation erforderlich ist. Dies kann bei geeigneten Umständen auch dann der Fall sein, wenn keine waben- oder gitterförmige Zwischenschicht **36** zur Verwendung kommt, oder wenn man auf die Deckschicht **34** verzichtet, Das Gemisch **10** polymerisiert nun bei Umgebungstemperatur, so dass man nach der vorgesehenen Zeit von etwa 8 Stunden den fertigen Formkörper **11** der Reaktor-Form **24** entnehmen kann. Für die weitere Verformung des Formkörpers **11** wird dieser mit einfachen Mitteln, beispielsweise einem Föhn oder einem Wärmeschrank, auf etwa 170 ° C bis 180 ° C erwärmt und anschliessend verformt. Dieser Verformungsvorgang kann wiederholt durchgeführt werden.

Fig. 4 zeigt einen Schnitt durch einen Formkörper **111** nach der Erfindung, welcher aus vier Matrixschichten **126a, 126b, 126c** und **126d** aufgebaut ist. Jede der Matrixschichten ist durch eine Schicht eingelegter Faserbahnen **128a, 128b, 128c** und **128d** verstärkt. Die Faserbahnen **128a** und **128c** einerseits und die Faserbahnen **128b** und **128d** anderseits verlaufen quer zueinander. Damit erreicht man, dass der Formkörper in zwei Richtungen hochbelastbar ist. Eine bessere Annäherung an einen in der Formkörperebene allseitig belastbaren, zweidimensional quasiisotropen Formkörper erhält man, wenn die Richtungen der Faserbahnen benachbarter Schichten jeweils nur um 45 Winkelgrad voneinander abweichen. Auf der obersten Matrixschicht **126d** ist eine an ihr haftende durchlässige Deckschicht **134** angebracht, welche die Aussenschicht des Formkörpers **111** bildet. Diese Deckschicht **134** dient zur Zufuhr von Sauerstoff bei der Polymerisation, zum Schutz der obersten Matrixschicht **126d** vor der Verwendung des Formkörpers **111** und zur Erzeugung einer Aufrauhung der obersten Matrixschicht **126d** bei der Entfernung der Deckschicht **134**, so dass sich das Anschleifen des Materials **111** vor einer Weiterverarbeitung durch Kleben erübrigt.

Die obige Beschreibung bezieht sich auf die Herstellung von Formkörpern in Plattenform. Formkörper in schwach gewölbter Form können in gleicher Weise hergestellt werden. Soll aber der Formkörper als komplizierteres geometrisches Gebilde erzeugt werden, so kann die Verdichtung der einzelnen Laminatschichten mittels einer in Längsrichtung flexiblen Leiste und die Verdichtung des gesamten Materials durch Bandagieren, beispielsweise mit einem Nylongewebe, erfolgen.

Die angegebenen Temperaturen, Drücke und Zeiten sind abhängig vom Kunststoff, aus dem die Matrixschichten hergestellt werden. Es sei noch einmal darauf hingewiesen, dass Polymerisationstemperaturen von 20 ° C bis 25 ° C günstig sind, weil bei Raumtemeratur gearbeitet werden kann. Ferner sind Temperaturen von weniger als 200 ° C für die Nachverformung günstig, da man diese Temperaturen in einfacher Weise, beispielsweise mit einem Heissluftföhn, erreicht.

Der neue Formkörper eignet sich besonders für orthopädische Geräte und wurde insbesondere zur Herstellung von Orthesen entwickelt. Er eignet sich aber auch für andere selbsttragende, insbesondere schalenförmige Elemente für orthopädische und andere Zwecke, wie beispielsweise für Auflagenflächen, insbesondere für lädierte Körperteile, oder für stutzende Sitz- und/oder Rückenflächen von Sesseln, Fahrzeugsitzen oder Rollstühlen, die dank der Nachverformbarkeit des Materials mit einfachen Hilfsmittel in situ an die jeweiligen Bedürfnisse ihrer Benutzer angepasst werden können. Ganz allgemein lässt er sich auch im Fahr- und Flugzeugbau, in der Raumfahrt und zur Herstellung von Prototypen verschiedener Gattungen verwenden.

## Patentansprüche

1. Plattenförmiger mehrschichtiger Formkörper, insbesondere für orthopädische Geräte, aus mindestens einer Matrixschicht aus amorphem thermoplastischen Polymer und einliegender Faserverstärkung,
   *dadurch gekennzeichnet, dass*
   das Polymer Polymethylmethacrylat ist, welches bei einer Temperatur zwischen 170 und 180°C thermisch wiederholt verformbar ist, und die Faserverstärkung aus Faserbahnen besteht, und der Formkörper mindestens eine äussere Deckschicht hat.

2. Formkörper nach Anspruch **1**,
   *daduch gekennzeicnet, dass*
   die Faserbahnen ausgewählt sind aus der Gruppe Gelege, Gestrick, Gewirke, Gewebe und Netz.

3. Formkörper nach Anspruch **1** oder **2**,
   *dadurch gekennzeichnet, dass*
   die Faserbahnen aus Kohlenstoff-Fasern bestehen.

4. Formkörper nach einem der voranstehenden Ansprüche,
   *dadurch gekennzeichnet, dass*
   die mindestens eine äussere Deckschicht eine perforierte Folie oder ein Gewebe ist.

5. Formkörper nach einem der voranstehenden Ansprüche,
*dadurch gekennzeichnet, dass*
die mindestens eine äussere Deckschicht vom Formkörper ablösbar ist.

6. Verfahren zur Herstellung eines plattenförmigen mehrschichtigen Formkörpers gemäss Anspruch **1**, mit den Schritten

   - Evakuieren eines Gemischs aus den unpolymerisierten Ausgangsstoffen des Polymethylmethacrylats,
   - Zugabe des Gemischs der Ausgangsstoffe auf oder in eine Reaktor-Form
   - Auflegen der Faserbahn auf das Gemisch,
   - Egalisieren und Verdichten des Gemischs zu einer ersten Matrixschicht,
   - wahlweises Wiederholen von mindestens einzelnen dieser Schritte für weitere Matrixschichten,
   - Aufbringen einer ablösbaren durchlässigen Deckschicht auf mindestens eine Aussenfläche der mindestens einen Matrixschicht,
   - Druckverdichten
   - Polymerisation der Monomeren in der mindestens einen Matrixschicht,

   wobei der Formkörper in der Reaktor-Form nach der Reaktorimprägnierung der Faserbahn mit den unpolymerisierten Bestandteilen durch deren Polymerisation in situ gebildet wird und
   wobei die weiteren Matrixschichten und die erste Matrixschicht mindestens teilweise ineinander übergehen und ein Laminat bilden..

7. Verfahren nach Anspruch **6**,
*dadurch gekennzeichnet, dass*
eine ebene oder gewölbte Reaktor-Form verwendet wird.

8. Verfahren nach Anspruch **6** bis **7**,
*dadurch gekennzeichnet, dass*
die Faserbahn ausgewählt ist aus der Gruppe Gelege, Gestrick, Gewirke, Netz und Gewebe.

9. Verfahren, nach Anspruch **6** bis **8**,
*dadurch gekennzeichnet, dass die*
Fasern der Faserbahn Kohlenstoff-Fasern sind.

10. Verfahren, nach Anspruch **6** bis **9**,
*dadurch gekennzeichnet, dass*
durch das Egalisieren und Verdichten die Faserbahn in das Gemisch gedrückt wird.

11. Verfahren nach Anspruch **6** bis **10**,
*dadurch gekennzeichnet, dass*
das Verdichten mit einer komplementär zur Reaktor-Form gestalteten und über die Form verschiebbaren Leiste durchgeführt wird.

12. Verfahren nach Anspruch **6** bis **11**,
*dadurch gekennzeichnet, dass*
die Polymerisation der Monomeren in einer sauerstoffhaltigen Umgebung durchgeführt wird.

13. Verfahren nach Anspruch **6** bis **12**,
dadurch gekennzeichnet, dass
die Polymerisation der Monomeren bei Raumtemperatur durchgeführt wird.

14. Verfahren nach Anspruch **6** bis **13**,
*dadurch gekennzeichnet, dass*
die Deckschicht flexibel ist, ausgewählt aus der Gruppe perforierte Folie und Kunststoffgewebe.

15. Verfahren nach Anspruch **6** bis **14**,
*dadurch gekennzeichnet, dass*
das Druckverdichten über eine zusätzliche waben- oder gitterartige Zwischenschicht ausgeübt wird.

16. Verfahren nach Anspruch **6** bis **15**,
*dadurch gekennzeichnet, dass*
das Druckverdichten mit einem Druck von 0,1 bis 0,5 bar durchgeführt wird.

17. Vorrichtung zur Durchführung des Verfahrens zur Herstellung eines
plattenförmigen mehrschichtigen Formkörpers nach einem der Ansprüche **6** bis **16**,
bestehend aus einer Reaktor-Form zur Aufnahme des Gemischs aus den unpolymerisierten Ausgangsstoffen, einer Einrichtung zum Egalisieren und Verdichten dieser Bestandteile und einem äusseren Druckkörper.

18. Vorrichtung nach Anspruch **17**,
*dadurch gekennzeichnet, dass*
die Einrichtung zum Egalisieren und Verdichten eine Leiste ist, die über die Form verschiebbar ist.

19. Vorrichtung nach Anspruch **17** oder **18**,
*dadurch gekennzeichnet, dass*
die Leiste an ihrem, zur Berührung mit dem Gemisch bestimmten Rand eine Bürste oder Lippe trägt.

20. Vorrichtung nach Anspruch **19**,
*dadurch gekennzeichnet, dass*
der Druckkörper druckseitig komplementär zur Form gestaltet ist.

21. Verwendung des plattenförmigen mehrschichtigen Formkörpers aus mindestens einerMatrixschicht aus amorphem thermoplastischen Polymer und einliegenden verstärkendenFasergebilden gemäss Anspruch **1** bis **5**
zur Herstellung von orthopädischen Geräten
durch Erwärmen bis zur plastischen Deformierbarkeit und nachfolgendes Verformen.

**Claims**

1. Plate-shaped formed part with multiple layers especially for orthopedic devices consisting of at least one matrix layer of amorphous, thermoplastic polymer with embedded reinforcing fibers,
wherein
the polymer is polymethyl methacrylate, which can repeatedly be thermoplastically redeformed at a temperature between 170° and 180°C, and the fiber-reinforcement consists of fiber breadth and the formed part has at least one external cover layer.

2. Formed part according to claim 1,
wherein
the fiber breadths are selected out of the group of layments in knitted woven or net form or of stichbonded breadths.

3. Formed part according to claim 1 or 2
wherein
the fiber breadths consist of carbon fiber.

4. Formed part according to one of the previous claims
wherein
the at least one external cover layer is formed by a perforated foil or woven material.

5. Formed part according to one of the previous claims
wherein
the at least one external cover layer is removable from the formed part.

6. Process for producing a plate-shaped formed part with the following steps:

   - Evacuation of a mixture of the unpolymerized reaction components of polymethyl methacrylate.
   - Adding the mixture of reaction components on or in a reactor-mold.
   - Placing a fiber breadth onto the mixture.
   - Evening out and compacting of the mixture to form a primary matrix layer.
   - Optional repetition of at least some of these steps to add additional matrix layers.
   - Placing a removable, permeable cover layer on at least one external area of the at least one matrix layer.
   - Compacting under pressure.

   - Polymerization of the monomeres in the at least one matrix layer where the formed part in the reactor-mold will be created in situ by its polymerization after the fiber structrure has been impregnated with the not yet polymerized components and
   where additional matrix layers and the primary matrix layer make at least partly transitions into each other and form a laminate-like material.

7. Process according to claim 6
wherein
a flat or curved reactor-mold is used.

8. Process according to claim 6 to 7
wherein
the fiber breadth selected out of the group of layments in knitted, woven and stichbonded breadths.

9. Process according to claim 6 to 8
wherein
the fiber of the fiber breadth is carbon fiber.

10. Process according to claim 6 to 9
wherein
the fiber breadth will be pushed into the mixture by evening out and compacting.

11. Process according to claim 6 to 10
wherein
the compacting will be done by a bar embodied complementary to the reactor-mold, wich can be displaced along the reactor-mold.

12. Process according to claim 6 to 11
wherein
the polymerization of the monomeres takes place in an oxygen-containing environment.

13. Process according to claim 6 to 12
wherein
the polymerization of the monomeres takes place at room temperature.

14. Process according to claim 6 to 14
wherein
the cover layer is flexible and was selected out of the group of perforated folio and woven plastic material.

15. Process according to claim 6 to 15
wherein
the compacting under pressure is done via an additional honeycomb- or grid-like intermediate layer.

16. Process according to claim 6 to 15
wherein
the compacting by pressure is done with 0,1 to 0,5 bar.

17. Device for executing the process of producing a plate-shaped formed part with multiple layers according to claims 6 to 16,
consisting of a reactor-mold receiving a mixture of unpolymerized reaction components, a device for evening out and compacting these components, a vacuum device and an external pressure element.

18. Device according to claim 17
wherein
the device for evening out and compacting is a bar, wich can be displaced along the reactor-mold.

19. Device according to claim 17 or 18
wherein
the bar has a brush or lip at the edge that is provided for contact with the mixture.

20. Device according to claim 19
wherein
the pressing-side of the pressure element is embodied complementary to the reactor-mold.

21. Method of using the plate-shaped formed part with multiple layers containing at least one matrix layer consisting of amorphous, thermoplastic synthetic material with embedded fortifying fiber structures according to claim 1 to 5 for the manufacturing of orthopedic devices by warming it up until plastically deformable and then shaping it.

**Revendications**

1. Corps façonné en forme de plaques à multiples couches avant tout pour des appareils orthopédiques,
composé au minimum d'une couche porteuse (matrice) d'un polymère amorphe et thermoplastique et d'un renforcement de fibres ci-inclus,
caractérisé en ce que

le polymère est un polyméthyle méthacrylate (PMMA) qui, à une température située entre 170 et 180°C, est thermoformable à plusieurs reprises
et que le renforcement de fibres consiste en différentes laizes de libres
et que le corps a au minimum une couche de recouvrement extérieure.

2. Corps selon la revendication 1
caractérisé en ce que les laizes de fibres sont selectionnées du groupe constitué par les tricots, tissus et filets ou par des fibres superposées et assemblées.

3. Corps selon l'une des revendications 1 ou 2
caractérisé en ce que

les laizes de libres consistent en libres de carbone.

4. Corps selon l'une des revendications précédentes
caractérisé en ce que

au minimum une couche de recouvrement extérieure est formée par une feuille perforée ou par un tissu.

5. Corps selon l'une des revendications précédentes
caractérisé en ce que

au minimum une couche de recouvrement extérieure est détachable du corps.

6. Procédé pour la fabrication d'un corps façonné en forme de plaques à plusieurs couches selon la revendication 1,
caractérisé par les pas suivants

- évacuation d'un mélange composé des matières de base non-polymérisées du polyméthyle méthacrylate

- adduction de ce mélange des matières, de base sur ou dans un moule-réacteur

- mise d'une laize fibreuse sur le mélange

- égalisation et condensation de ce mélange afin de former une première couche porteuse (matrice)

- répétition facultative de - au minimum - quelques de ces pas afin d'obtenir d'autres couches porteuses (matrices)

- application d'une couche de recouvrement détachable et perméable sur - au minimum - une surface extérieure des - au minimum une - couches porteuses existentes

- condensation par pression

- polymérisation des monomères dans - au minimum -une couche porteuse.
Pendant ce procédé,
le corps façonné est creé par la polymérisation dans le moule-réacteur in situ après l'imprégnation des laizes de fibres avec les composants pas encore polymérisés, et les autres couches porteuses se confondent au moins partialement avec la première couche porteuse et forment un laminage.

**7.** Procédé selon la revendication 6
caractérisé en ce que

le moule-réacteur est plat ou bombée.

**8.** Procédé selon l'une des revendications 6 à 7
caractérisé en ce que

la laize de fibres est selectionné du groupe
constitué par les tricots, tissus et filets ou par
des fibres superposées et assemblées.

**9.** Procédé selon l'une des revendications 6 à 8
caractérisé en ce que

les fibres de la laize fibreuse sont des fibres de
carbone.

**10.** Procédé selon l'une des revendications 6 à 9
caractérisé en ce que

par moyen de l'égalisation et la condensation
la laize de fibres est poussée dans le mélange.

**11.** Procédé selon l'une des revendications 6 à 10
caractérisé en ce que

la condensation s'effectue
par moyen d'un listel ayant une forme complémentaire à celle du moule-réacteur et coulissant sur le moule-réacteur.

**12.** Procédé selon l'une des revendications 6 à 11
caractérisé en ce que

la polymérisation des monomères s'effectue
dans un entourage oxygéré.

**13.** Procédé selon l'une des revendications 6 à 12
caractérisé en ce que

la polymérisation des monomères s'effectue à
la température ambiante.

**14.** Procédé selon l'une des revendications 6 à 13
caractérisé en ce que

la couche de recouvrement est flexible et
selectionnée du groupe des feuilles perforées
et des matières plastiques.

**15.** Procédé selon l'une des revendications 6 à 14
caractérisé en ce que

la condensation par pression s'effectue moyennant une autre couche intermédiaire en forme
de rayon ou de grille.

**16.** Procédé selon l'une des revendications 6 à 15
caractérise en ce que

la condensation par pression s'effectue sous
une pression de 0,1 à 0,5 bar.

**17.** Dispositif façonné pour la réalisation du procédé
ayant pour but la fabrication d'un corps façonné en
forme de plaques à plusieurs couches selon l'une
des revendications 6 à 16,
consistant en un moule-réacteur ayant la fonction
de recevoir le mélange des matières de base non-
polymérisées, en une installation ayant la fonction d
égaliser et de condenser ces composants et en un
corps de pression extérieur.

**18.** Dispositif selon la revendication 17
caractérisé en ce que

l'installation ayant la fonction d'égaliser et de
condenser est un listel qui coulisse sur la
forme.

**19.** Dispositif selon l'une des revendications 17 ou 18
caractérisé en ce que

le listel est pourvu d'une brosse ou d'un lèvre
au bord destiné à toucher le mélange.

**20.** Dispositif selon la revendication 19
caractérisé en ce que

l'élément de pression est construit complémentairement au moule-réacteur à ce côté où sont
effectives les forces de pression.

**21.** Utilisation du corps façonné en forme de plaques et
à plusieurs couches composé au minimum d'une
couche porteuse (matrice) de polymères amorphes
et thermoplastiques et d'une matière fibreuse renforçante ci-incluse selon l'une des revendications 1
à 5
pour la fabrication d'appareillages orthopédiques
par moyen du chauffage jusqu'à la déformabilité
plastique et la transformation ultérieure.

### Fig. 1

### Fig. 4

### Fig. 2

### Fig. 3